(19) Europäisches Patentamt
European Patent Office
Office européen des brevets

(11) Numéro de publication: **0 166 652**
**B1**

(12) **FASCICULE DE BREVET EUROPEEN**

(45) Date de publication du fascicule du brevet: **25.04.90**

(21) Numéro de dépôt: **85401187.1**

(22) Date de dépôt: **14.06.85**

(51) Int. Cl.$^5$: **C 07 D 471/04,**
C 07 D 233/70,
C 07 D 487/04, C 07 D 513/04
// C07D277/46 ,(C07D471/04,
235:00, 221:00),(C07D487/04,
239:00, 235:00),(C07D487/04,
235:00, 235:00),(C07D513/04,
277:00, 235:00)

(54) **Procédé d'ouverture du cycle des gem dicyanoépoxydes et nouveaux composés obtenus par ce procédé.**

(30) Priorité: **15.06.84 FR 8409434**

(43) Date de publication de la demande:
**02.01.86 Bulletin 86/01**

(45) Mention de la délivrance du brevet:
**25.04.90 Bulletin 90/17**

(84) Etats contractants désignés:
**AT BE CH DE FR GB IT LI LU NL SE**

(56) Documents cités:

**JOURNAL OF ORGANIC CHEMISTRY, vol. 43, no. 19. 15 septembre 1978, pages 3732-3736, American Chemical Society, Washington, US; M. BAUDY et al.: "Reaction of N-substituted thioamides with gem-dicyano epoxides: a new synthetic route to anhydro-4-hydroxythiazolium hydroxides"**

(73) Titulaire: **CENTRE NATIONAL DE LA RECHERCHE SCIENTIFIQUE (CNRS)**
**15, Quai Anatole France**
**F-75007 Paris (FR)**

(72) Inventeur: **Robert, Albert**
**15, rue Courteline**
**F-35000 Rennes (FR)**
Inventeur: **Jaguelin Sylvie**
**7, rue de Douves**
**F-35600 Redon (FR)**
Inventeur: **Guinamant, Jean-Luc**
**Karret Bolazec**
**F-29216 Plougonven (FR)**

(74) Mandataire: **Jolly, Jean-Pierre et al**
**Cabinet BROT et JOLLY 83, rue d'Amsterdam**
**F-75008 Paris (FR)**

**EP 0 166 652 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

(56) Documents cités:

INTERNATIONAL JOURNAL OF METHODS IN SYNTHETIC ORGANIC CHEMISTRY, 1976, pages 261-262, Georg Thieme Verlag, Stuttgart, DE; M. FERREY et al.: "A new synthesis of 4-hydroxythiazoles"

INTERNATIONAL JOURNAL OF METHODS IN SYNTHETIC ORGANIC CHEMISTRY, no. 7, juillet 1981, pages 981-984, Georg Thieme Verlag, Stuttgart, DE; M. BAUDY-FLOC'H et al.: "A general synthesis of ring-fused mesoionic thiazolines from 2,2-dicyanooxiranes under neutral conditions"

**Description**

La présente invention concerne un procédé de synthèse de dérivés thiazoles et imidazoles et d'autres composés hétérocycliques, à partir de gem dicyanoépoxydes.

on sait que la réaction des époxydes avec des hydracides provoque l'ouverture du cycle époxyde et conduit à des chlorhydrines généralement stables.

L'ouverture des cycles époxydes des gem dicyanoépoxydes par des dérivés thiocarbonylés a également fait l'objet de diverses études ("Obtention d'intermédiaires tétrahédriques au cours de la réaction des gem dicyanoépoxydes avec les thioamides substitués. Evolution de ces intermédiaires en thiazoles mésoioniques", M. BAUDY et A. ROBERT, Tetrahedron Letters, Vol. 21, p. 2517—2520, 1980; voir également "A general synthesis of ring-fused mesoionic thiazolines from 2,2-dicyano-oxyranes under neutral conditions", M. BAUDY-FLOC'H, A. ROBERT, Synthesis, N° 12, December 1981, p. 981—984).

En poursuivant leur étude de l'ouverture du cycle des gem dicyanoépoxydes, les inventeurs ont établi que ces composés sont susceptibles de réagir avec des composés mono ou binucléophiles en présence d'un hydracide, pour conduire à diverses familles de composés, notamment des composés arylacétiques ou arylpropioniques, lorsque le réactif est mono-nucléophile, et à des composés hétérocycliques, notamment de la famille des thiazoles et des imidazoles, lorsque le réactif est binucléophile.

Un but de l'invention est donc de proposer un procédé d'ouverture du cycle de gem dicyanoépoxydes de formule

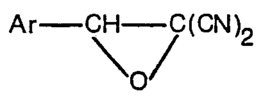

en vue de l'obtention des dérivés mennés ci-dessus.

A cet effet, l'invention a pour objet un procédé d'ouverture du cycle des gem dicyanoépoxydes de formule

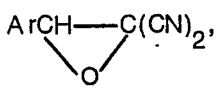

où Ar désigne un groupe aryle, dans lequel on fait réagir un gem dicyanoépoxyde avec un hydracide dans des conditions usuelles dans la technique, caractérisé en ce qu'un composé binucléophile azoté est présent dans le milieu réactionnel.

Le procédé conforme à l'invention consiste donc à faire réagir en une étape, de façon sélective, deux réactifs nucléophiles différents -l'hydracide et un second nucléophile- sur des époxydes substitués par deux groupes partants cyano. En effet, contrairement à l'ouverture classique des époxydes par les hydracides, qui conduit à des chlorydrines stables, les inventeurs ont établi, mais sans que la validité et la portée de la présente invention soient liées à cette théorie, que les gem dicyanoépoxydes réagissent avec les hydracides pour donner des cyanhydrines instables, qui évoluent instantanément en des intermédiaires particulièrement intéressants, puisque substitués par un groupe cyanoformyle, qui, on le sait, sont des composés très réactifs. Le second nucléophile présent dans le milieu réagit immédiatement avec cet intermédiaire pour conduire avec de très bons rendements à un grand nombre de composés, dont certains sont nouveaux.

On a déjà décrit, dans la technique antérieure, l'ouverture du cycle des gem dicyanoépoxydes par des réactifs binucléophiles possédant un groupe thiocarbonylé (J. Org. Chem. 1978, 43, 3732; Synthesis, 1976, p. 261—262; Synthesis, 1981, p. 981—984). Les réactions décrites sont cependant différentes de celles de la présente invention, car elles sont conduites en l'absence de tout hydracide ou acide faiblement nucléophile et consistent en une ouverture directe de l'époxyde par attaque nucléophile du groupement thiocarbonyle.

Au contraire, dans le cadre du présent procédé, en présence d'un hydracide ou d'un acide faiblement nucléophile, un dérivé de type cyanoformyle α-halogéné se forme dans une première étape et c'est ce composé très réactif, qui réagit in situ avec le réactif binucléophile présent dans le milieu.

Cette méthode de synthése de très nombreux composés hétérocycliques se caractérise par une double sélectivité:

a) C'est toujours l'hydracide qui attaque l'époxyde dans une première étape et l'halogène se fixe exclusivement en β des groupes cyano.

b) C'est toujours le site le moins basique du réactif binucléophile qui attaque dans une seconde étape le dérivé cyanoformyle formé in situ.

Cette réactivité remarquable permet d'expliquer les rendements généralement élevés des synthèse réalisées selon ce procédé.

Il faut souligner que les composés hétérocycliques décrits dans la suite de la présente demande ne peuvent pas être obtenus en milieu neutre selon le procédé décrit dans les références J. Org. Chem. 1978, 43, 3732; Synthesis 1976, p. 261; Synthesis 1981, p. 981.

Les inventeurs ont donc établi que l'intermédiaire halogéné substitué par un groupe cyanoformyle résultant de l'ouverture des gem dicyanoépoxydes réagit avec le composé bi-nucléophile pour conduire à un composé hétérocyclique.

Il est possible de préparer par le procédé de l'invention un imidazothiazole en utilisant un composé binucléophile dans lequel l'atome de carbone séparant les deux atomes d'azote est lié à un atome de soufre.

Dans le cas où l'un des atomes de carbone de l'époxyde porte un radical R, notamment un radical aryle, et un atome d'hydrogène, et si l'on représente par Nu Nu le composé binucléophile, la réaction globale peut donc être représentée par le schéma suivant:

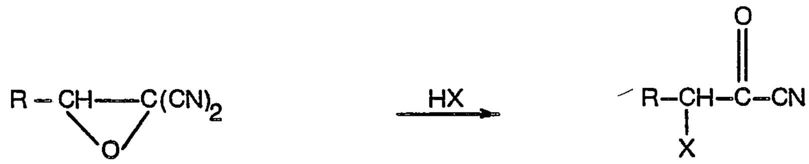


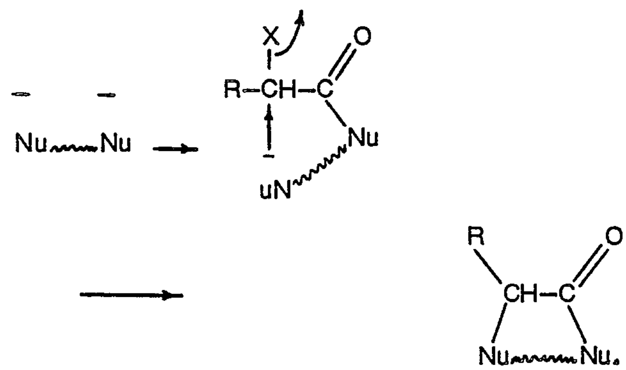

Ce procédé s'applique en particulier à la préparation de nombreux composés de la série des thiazoles et des imidazoles, dont l'importance industrielle est considérable.

On va maintenant décrire en détail différentes formes de mise en oeuvre de l'invention. Il est ententu que cette description ne vise qu'à illustrer l'invention et qu'elle n'a pas de caractère limitatif.

Les gem di-cyano époxydes de départ peuvent être facilement obtenus par les réactions successives suivantes, qui sont bien connues dans la technique:

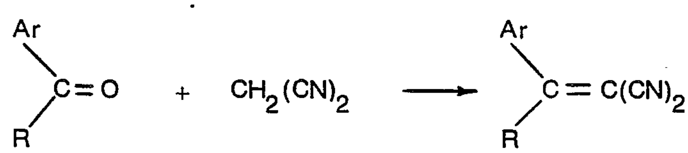

*1*

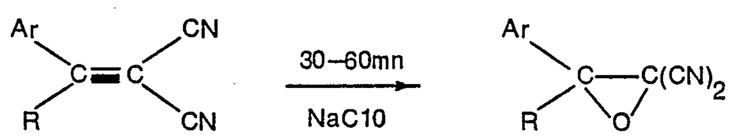


Préparation des imidazopyridines

L'Exemple 1 ci-après décrit la synthèse de diverses imidazopyridines:

Exemple 1

1a — *Synthèse de l'imidazopyridine*

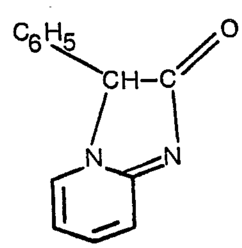

1,7 g d'époxyde

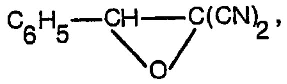

1,3 g de chlorhydrate de 2-aminopyridine en solution dans 60 $cm^3$ d'acétonitrile sont portés à reflux pendant 4 heures. L'imidazo (1—2, a) pyridine précipite après addition de triéthylamine. F = 250°C (acétone), Rdt 64%, masse $C_{13}H_{10}N_2O$ Calc. 210,0793; tr. 210,078; RMN ($CDCl_3$ $CF_3CO_2H$) δ (ppm): 6,20 (s, 1H), 6,90—8,40 (multiplet, 1 OH). Ce composé présente les mêmes caractéristiques que celui préparé dans la littérature selon une méthode différente.

1b — *Synthèse des imidazo (1,2-a) pyridines*

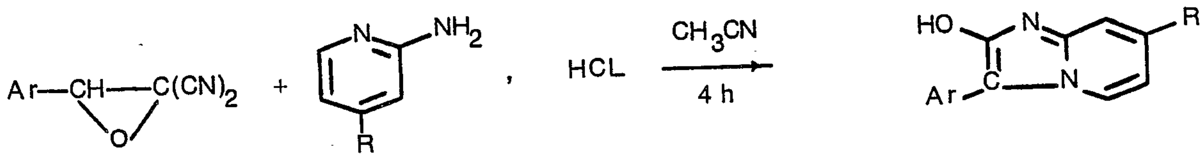


A la connaissance des inventeurs, ces imidazo (1,2-a) pyridines sont des composés nouveaux, sauf lorsque Ar et R désignent respectivement et simultanément $C_6H_5$ et H. En effet, il s'agit de composés du type aryl-3 imidazo (1,2-a) pyridine-ol 2, qui sont différents des aryl-2 imidazo (1,2-a) pyridine-ol 3 décrits dans J. Het. Chem. Vol. 16, 187 (1979) et Aust. J. Chem. 1981, 34(6), 1295—1302 (selon le Chem. Abst. Vol. 95, p. 645, 187153 d).

On porte à reflux $10^{-2}$ mole d'époxyde et $10^{-2}$ mole de chlorhydrate de 2 aminopyridine dans 60 $cm^3$ d'acétonitrile pendant 4 h. Les imidazo (1,2-a) pyridines précipitent après addition de $NEt_3$. Ces composés sont caractérisés par les données du tableau I. L'imidazo (1,2-a) pyridine (Ar = Ph, R = H) présente les mêmes caractéristiques que l'imidazo (1,2-a) pyridine (Ar = Ph, R = H) décrit dans la littérature et préparé selon une autre méthode.

1c — *Synthèse des imidazo (1,2-a) pyridines mésoioniques*

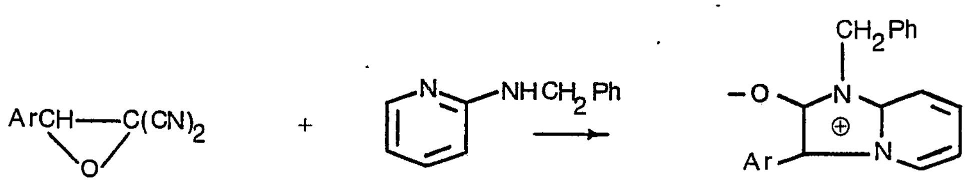

Les imidazo (1,2-a) pyridines mésoioniques ainsi préparées sont des composés nouveaux. En effet, il s'agit d'imidazo (1,2-a) pyrimidine-ol-2, différentes des imidazo (1,2-a) pyrimidine-ol-3 décrites dans Aust. J. Chem. 1981, 34 (6), 1295—1302 (selon Chem. Aust. Vol. 95, p 645, 187153 d).

Le mode opératoire de la réaction est identique au précédent (1b ci-dessus).

Caractéristiques du composé obtenu pour Ar = $pNO_2C_6H_4$: Rdt: 60%, F = 262°C, formule $C_{20}H_{15}N_3O_3$.

Masse calculée: 345,1113; trouvée: 345,110.

RMN ($CDCl_3$, $CF_3CO_2H$) 5,17 (s, 2H); 7,05 à 7,65 (m, 9H); 8,05 à 8,50 (m, 4H).

IR (Nujol) $\nu_{co}$ 1639.

Le Tableau I ci-après rassemble les caractéristiques de diverses imidazo (1,2-a) pyridines préparées par le procédé selon l'invention:

TABLEAU I

Imidazo (1,2-a) pyridines

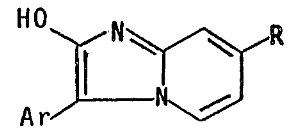

| R | Ar | Rdt % | θf°C | Formule | Masse $M^+$ | ANALYSE C | H | N | Cl | RMN $CDCl_3+CF_3CO_2H$ a) | IR (Nujol) |
|---|---|---|---|---|---|---|---|---|---|---|---|
| H | $C_6H_5$ | 64 | 250 | $C_{13}H_{10}N_2O$* | Th.210,07930<br>Tr.210,0783 | 74,27<br>74,04 | 4,79<br>4,71 | 13,32<br>13,08 | | 6,20(s, 1H)<br>690 à 8,40(m,10H | 3362 $cm^{-1}$ (L)<br>1633 $cm^{-1}$ |
| H | $pClC_6H_4$ | 50 | 255 | $C_{13}H_9N_2OCl$* | Th.244,0403<br>Tr. 244,0392 | 63,81<br>63,83 | 3,70<br>3,67 | 11,44<br>11,50 | 14,48<br>14,40 | 6,20(s, 1H)<br>7,10-8,57(m,8H) | 3364 $cm^{-1}$ (L)<br>1636 $cm^{-1}$ |
| H | $pNO_2C_6H_4$ | 52 | 300 | $C_{13}H_9N_3O_3$* | Th.255,0643<br>Tr.255,0646 | 61,17<br>61,04 | 3,55<br>3,47 | 16,46<br>16,37 | | 6,62 (s, 1H)<br>7,50-8,80(m,8H) | 3360 $cm^{-1}$ (L)<br>1634 $cm^{-1}$ |
| H | $pCH_3C_6H_4$ | 52 | 252 | $C_{14}H_{12}N_2O$* | Th.224,0949<br>Tr.224,0945 | 74,98<br>75,05 | 5,39<br>5,36 | 12,49<br>12,47 | | 2,27 (s,3H)<br>6,90-8,50(m,SH) | 3359 $cm^{-1}$ (L)<br>1632 $cm^{-1}$ |
| $CH_3$ | $pClC_6H_4$ | 50 | 274 | $C_{14}H_{11}N_2OCl$* | Th.258,0559<br>Tr.258,0563 | 65,00<br>64,88 | 4,28<br>4,27 | 10,83<br>10,74 | 13,70<br>14,11 | 2,45(s,3H)6,20<br>(s,1H)<br>7,00-8,30(m,7H) | 3363 $cm^{-1}$ (L)<br>1645 $cm^{-1}$ |
| $CH_3$ | $pNO_2C_6H_4$ | 53 | 300 | $C_{14}H_{11}N_3O_3$* | Th.269,0800<br>Tr.269,0792 | 62,45<br>62,28 | 4,11<br>4,04 | 15,60<br>15,66 | | 2,70 (s, 3H)<br>6,50 (s, 1H)<br>7,32-8,52(m,7H) | 3362 $cm^{-1}$<br>1641 $cm^{-1}$ |
| $CH_3$ | $pCH_3C_6H_4$ | 49 | 268 | $C_{15}H_{14}N_2O$* | Th.238,1106<br>Tr.238,110 | 75,60<br>75,43 | 5,92<br>5,93 | 11,75<br>11,46 | | 2,30(s,3H)<br>250(s, 3H) OH<br>6,00(s,1H)6,85-<br>8,00(m,7H)C=N | 3360 $cm^{-1}$ (L)<br>1638 $cm^{-1}$ |
| H | $mNO_2C_6H_4$ | | 300 | $C_{13}H_9N_3O_3$* | Th.255,06438<br>Tr.255,0644 | 61,17<br>61,06 | 3,55<br>3,42 | 16,46<br>16,52 | | 6,57 (s,1H)<br>7,10 à 8,75(m,8H | 3350 $cm^{-1}$<br>1633 $cm^{-1}$ |

a) Forme tautomère oxo. * composé nouveau

2 — *Synthèse des imidazo pyrimidines*

Le schéma réactionnel est le suivant:

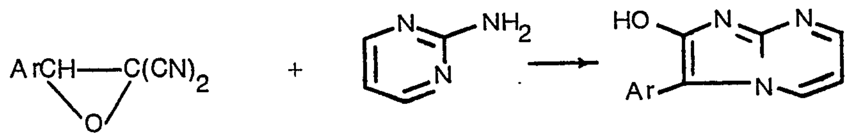

Imidazo pyrimidines

Le mode opératoire précédent est utilisé mais les rendements sont relativement faibles (20 à 30%).

| | | |
|---|---|---|
| AR = $pClC_6H_4$ | F = 260°C | Masse th. 245,0355 |
| | | „ tr. 245,035 |
| Ar = $pCH_3C_6H_4$ | F = 272°C | „ th. 256,096 |
| | | „ tr. 256,059 |
| Ar = $pNO_2C_6H_4$ | F = 300°C | „ th. 225,0902 |
| | | 225,090 |

Exemple 2

3 — *Synthèse des hydroxy-4 imidazoles*

Le schéma réactionnel est le suivant:

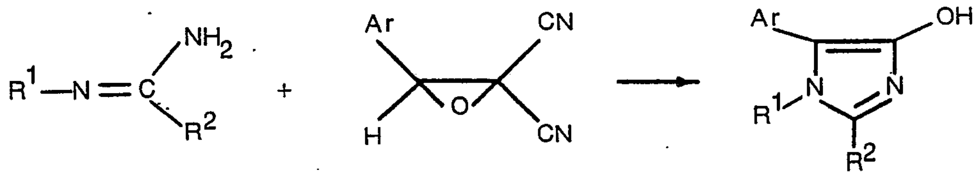

A la connaissance des inventeurs, les hydroxy-4 imidazoles obtenus sont des composés nouveaux.

On dissout dans 50 ml d'acétonitrile $10^{-2}$ mole d'époxyde et on ajoute sous agitation $10^{-2}$ mole de monohydrate d'amidine. Après 30 mn de réaction, on filtre le précipité formé sur büchner et on lave par 10 ml d'acétonitrile (tableau VII).

Les rendements sont relativement faibles (20 à 30%).

Le Tableau II ci-après donne les caractéristiques de divers hydroxy-4 imidazoles ainsi préparés:

TABLEAU II

Hydroxy-4 imidazoles.

| Ar | $R^1$ | $R^2$ | θf °C | Formule | Masse th / tr | RMN δppm $CDCl_3+CF_3CO_2H$ | IR Nujol |
|---|---|---|---|---|---|---|---|
| $C_6H_5$ | $C_6H_5$ | $C_6H_5$ | 278 d | $C_{21}H_{16}N_2O$ * | 312,1262<br>312,125 | 7,00 à 7,65 | 1602<br>3360 (L) |
| $pClC_6H_4$ | $C_6H_5$ | $C_6H_5$ | >300 | $C_{21}H_{15}N_2OCl$* | 346,0872<br>346,086 | 7,00 à 7,70 | 1600<br>3350 (L) |
| $pMeC_6H_4$ | $C_6H_5$ | $C_6H_5$ | 282 d | $C_{22}H_{18}N_2O$* | 326,1419<br>326,141 | 6,90 à 7,70 | 1604<br>3420 (L) |
| $pNO_2C_6H_4$ | $C_6H_5$ | $C_6H_5$ | >300 | $C_{21}H_{15}N_3O_3$* | 357,1113<br>357,111 | 7,15 à 7,65<br>8,05 à 8,25 | 1585<br>3400 (L) |

*composé nouveau

Exemple III

4 — *Synthèse des imidazo imidazoles*

Le processus réactionnel est le suivant

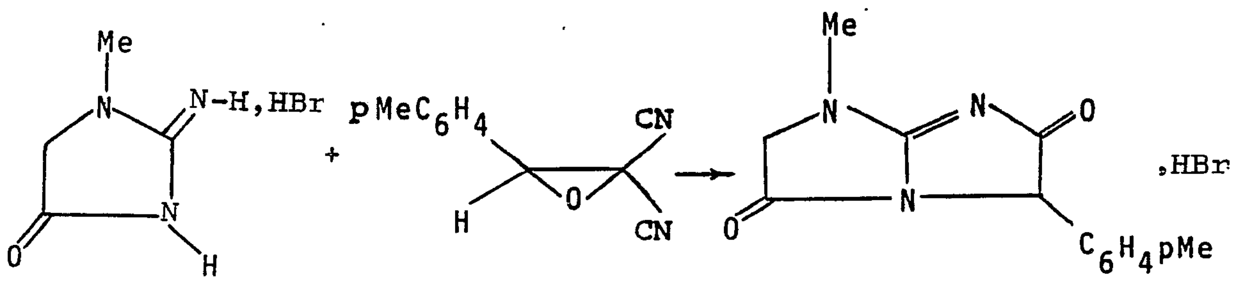

Dans un bécher, on ajoute dans l'ordre:

— 50 $cm^3$ d'acétonitrile,

— $10^{-2}$ mole de bromhydrate de créatinine

— $10^{-2}$ mole d'époxyde (Ar = $pCH_3C_6H_4$)

On laisse sous agitation pendant 12 h, puis on filtre sur büchner et on lave à l'acétonitrile (20 ml).

| Rdt % | F | Formule | Masse |
|---|---|---|---|
| 50 | 172 d | $C_{13}H_{13}N_3O_2$ | th. 243,1007<br>tr. 243,099 |

RMN ($CDCl_3$ + $CF_3CO_2H$) ppm 2,40 (s, 3H), 3,20 (m, 3H), 4,25 (m, 2H), 5,40 (s, 1H), 7,00 à 7,70 (m, 4H). IR (Nujol) $cm_{-1}$: $\nu_{CO}$ = 1753, 1705.

Exemple IV

5 — *Synthèse des imidazothiazoles et des imidazothiazolines*

Dans ce cas, les dérivés halogénés intermédiaires ont pu être isolés. Ces halogéno thiazolines sont des composés nouveaux.

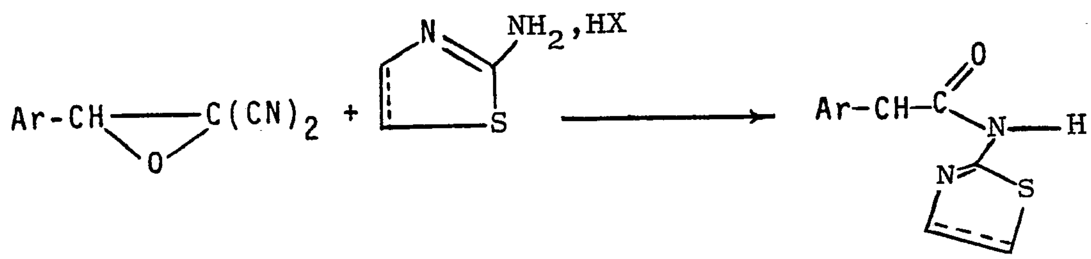

(les pointillés dans le cycle thiazole symbolisent la possibilité d'une double liaison).

Ces thiazolines ou thiazoles intermédiaires sont préparés en faisant réagir $10^{-2}$ mole de bromhydrate (ou chlorhydrate) d'amino-2 thiazoline avec $10^{-2}$ mole d'époxyde dans 30 $cm^3$ d'acétonitrile pendant 2 h à la température ambiante. Les principales caractéristiques des halogéno-thiazolines sont rassemblées dans le tableau III ci-après.

Les caractéristiques du thiazole

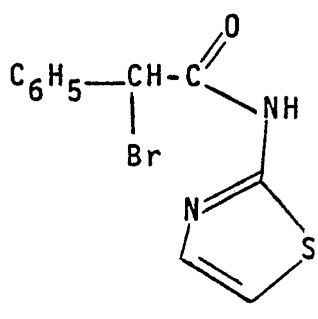

sont les

Rdt: 40%, F = 191°C (EtOH). $C_{11}H_9N_2OSBr$ Masse th. 297,9595

tr. 297,960

RMN ($CDCl_3$, $CF_3CO_2H$) 5,70 (1H, s) 7,50 (7H, m). IR (Nujol) 1670 $cm^{-1}$

TABLEAU III

Halogénothiazolines 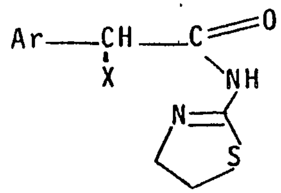

| Ar | X | Rdt % | θf°C | Formule | Masse $M^+$ | exp th | RMN δ ppm $CDCl_3 + CF_3CO_2H$ | IR $cm^{-1}$ (Nujol) |
|---|---|---|---|---|---|---|---|---|
| $C_6H_5$ | Br | 95 | 130 | $C_{11}H_{11}N_2OSBr$* | 297,9778<br>297,9775 | | 3,50 : 2H (t) 4,15 : 2H (t)<br>5,60 : 1H (s) 7,40 : 5H (m) | 3250<br>1625 |
| $pClC_6H_4$ | Br | 95 | 146 | $C_{11}H_{10}N_2OSBrCl$* | 331,9390<br>331,9386 | | 3,55 : 2H (t) 4,20 : 2H (t)<br>5,55 : 1H (s) 7,40 : 4H (m) | 3150<br>1627 |
| $pCH_3C_6H_4$ | Br | 90 | 176 | $C_{12}H_{13}N_2OSBr$* | 311,9936 | | 3,40 : 2H (t) 4,10 : 2H (t)<br>2,30 : 3H (s)<br>5,60 : 1H (s) 7,30 : 4H (m) | 3147<br><br>1628 |
| $pNO_2C_6H_4$ | Br | 80 | 146 | $C_{11}H_{10}N_3O_3SBr$* | | | 3,60 : 2H (t) 4,25 : 2H (t)<br>5,80 : 1H (s) 8,00 : 4H (m) | 3300<br>1618 |
| $C_6H_5$ | Cl | 70 | 178 | $C_{11}H_{11}N_2OSCl$* | 254,0279<br>254,0281 | | 3,60 : 2H (t) 4,20 : 2H (t)<br>5,60 : 1H (s) 7,45 : 5H (m) | 3250<br>1618 |
| $pClC_6H_4$ | Cl | 70 | 163 | $C_{11}H_{10}N_2OSCl_2$* | 287,9915<br>287,9891 | | 3,55 : 2H (t) 4,20 : 2H (t)<br>5,60 : 1H (s) 7,40 : 4H (m) | 3230<br>1640 |
| $pCH_3C_6H_4$ | Cl | 75 | 163 | $C_{12}H_{13}N_2OSCl$* | 268,0424<br>268,0437 | | 3,60 : 2H (t) 4,10 : 2H (t)<br>5,60 : 1H (s) 7,30 : 4H (m)<br>2,35 : 3H (s) | 3250<br>1630 |
| $pNO_2C_6H_4$ | Cl | 60 | 167 | $C_{11}H_{10}N_3O_3SCl$* | 299,0108<br>299,0131 | | 3,60 : 2H (t) 4,20 : 2H (t)<br>5,75 : 1H (s) 8,00 : 4H (m) | 3250<br>1628 |

*composé nouveau

6 — *Cyclisation des thiazolines halogénées précédentes*

Les thiazolines halogénées évoluent en imidazothiazolines après 18 h. d'ébullition dans du benzène.

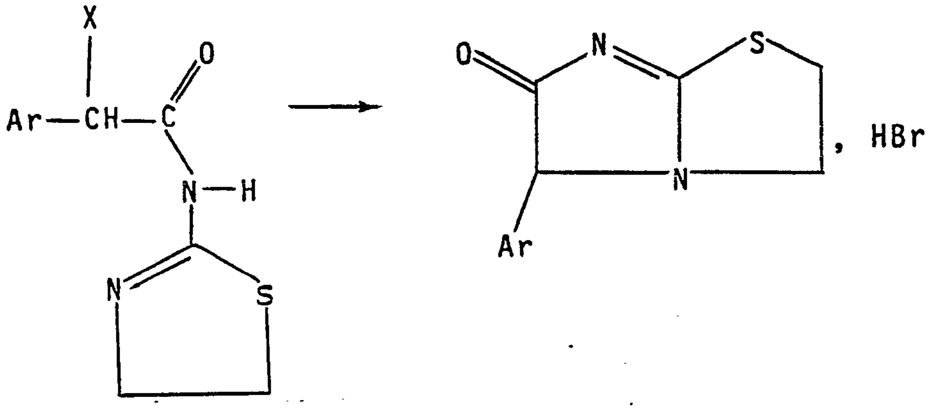

Les imidazothiazolines obtenues sont des composés nouveaux.

Le Tableau IV ci-après rassemble les caractéristiques des imidazothiazolines ainsi préparées.

TABLEAU IV

Imidazothiazolines

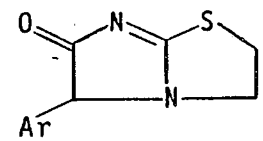

, HBr

| Ar | Rdt % | θf°C | Formule | Masse M+ exp. / th. | RMN ($CDCl_3$ + $CF_3CO_2H$) δ ppm | IR $cm^{-1}$ Nujol |
|---|---|---|---|---|---|---|
| $C_6H_5$ | 85 | 214 | $C_{11}H_{10}N_2OS$* | 218,0518<br>218,0514 | 3,50 = 2H (t) 4,10 : 2H (t)<br>6,00 = 1H (s) 7,35 : 5H (m) | ν C=0 : 1755 |
| $pCH_3C_6H_4$ | 80 | 195 | $C_{12}H_{12}N_2OS$* | 232,0666<br>232,0670 | 2,30 : 3H (s) 3,40 : 2H (t) 4,10:2H(t)<br>5,60 : 1H (s) 7,30 : 4H (m) | ν C=0 : 1747 |

* composé nouveau

7 — *Synthèse des hydroxy imidazo hydroxythiazoles*

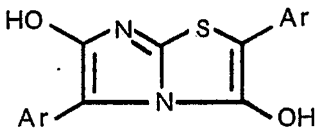

Les gem dicyanoépoxydes réagissent avec les halohydrates d'amino-2 thiazolones-4 au reflux de l'acétonitrile (3 h).

Exemple:

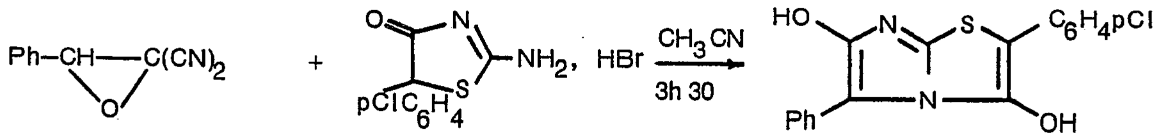


F: 300°C (EtOH);

$C_{17}H_{11}N_2O_2SCl$ Masse $M^+$ Calc. 342,0230
Tr. 342,021

RMN ($CDCl_3$, $CF_3CO_2H$) 7,50 (m)

IR (Nujol): 3250 $cm^{-1}$; 1658 $cm^{-1}$

Le procédé d'ouverture du cycle des gem dicyano époxydes qui fait l'objet de la présente invention se révèle donc être un procédé original et simple pour la synthèse d'innombrables composés, connus ou non.

**Revendications**

1. Procédé d'ouverture du cycle des gem dicyanoépoxydes de formule

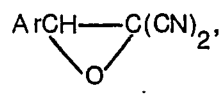

où Ar désigne un groupe aryle, dans lequel on fait réagir un gem dicyanoépoxyde avec un hydracide dans des conditions usuelles dans la technique, caractérisé en ce qu'un composé binucléophile azoté est présent dans le milieu réactionnel.

2. Procédé selon la revendication 1, caractérisé en ce que ledit composé binucléophile est une amidine et le produit obtenu est un hydroxy-4 imidazole de formule:

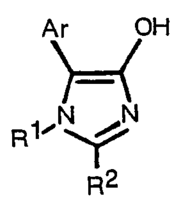

où Ar désigne un groupe aryle et $R^1$ et $R^2$ désignent chacun un groupe phényle.

3. Procédé selon la revendication 1, caractérisé en ce que ledit composé binucléophile est un amino-2 thiazole et le produit obtenu est un oxo-6-aryl-5 imidazothiazole.

4. Procédé selon la revendication 1, caractérisé en ce que ledit composé binucléophile est une amino-2 pyridine et le produit obtenu est un imidazo (1,2,a) pyridine de formule:

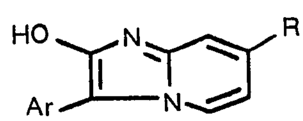

où Ar désigne un groupe aryle et R un hydrogène ou un méthyle.

5. Procédé selon la revendication 1, caractérisé en ce que ledit composé binucléophile est la N-benzylamino-2 pyridine et le produit obtenu est une imidazo (1,2-a) pyridine mésoionique de formule:

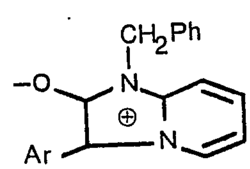

où Ar désigne un groupe aryle.

6. Procédé selon la revendication 1, caractérisé en ce que ledit composé binucléophile est l'amino-2 pyrimidine et le produit obtenu est une imidazopyrimidine de formule:

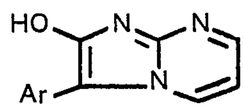

où Ar désigne un groupe aryle.

**Patentansprüche**

1. Verfahren zur Ringöffnung der gem Dicyanoepoxide der Formel

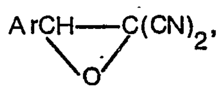

worin Ar eine Arylgruppe bedeutet, in welchem ein gem Dicyanoepoxid mit einem Hydracid unter in der Technik üblichen Reaktionsbedingungen umgesetzt wird, dadurch gekennzeichnet, daß eine binucleophil azotierte Verbindung in dem Reaktionsmedium vorhanden ist.

2. Verfahren nach Anspruch 1, dadurch gekennzeichnet, daß die binucleophile Verbindung ein Amidin ist und das erhaltene Produkt ein 4-Hydroxyimidazol der Formel

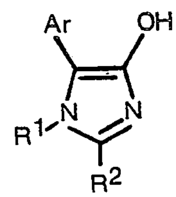

ist, worin Ar eine Arylgruppe bedeutet und $R^1$ und $R^2$ jeweils eine Phenylgruppe bedeuten.

3. Verfahren nach Anspruch 1, dadurch gekennzeichnet, daß die binucleophile Verbindung ein 2-Aminothiazol ist und die erhaltene Verbindung ein 6-Ozo-5-arylimidazothiazol ist.

4. Verfahren nach Anspruch 1, dadurch gekennzeichnet, daß die binucleophile Verbindung ein 2-Aminopyridin ist und das erhaltene Produkt ein Imidazo[1,2-a]pyridin der Formel

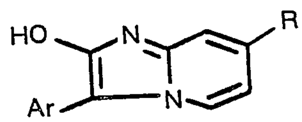

ist, worin Ar eine Arylgruppe bedeutet und R Wasserstoff oder Methyl bedeutet.

5. Verfahren nach Anspruch 1, dadurch gekennzeichnet, daß die binucleophile Verbindung N-Benzyl-2-aminopyridin ist und das erhaltene Produkt ein mesoionisches Imidazo/1,2-a/pyridin der Formel

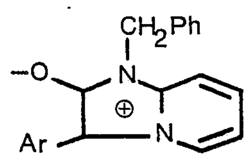

ist, worin Ar eine Arylgruppe bedeutet.

6. Verfahren nach Anspruch 1, dadurch gekennzeichnet, daß die binucleophile Verbindung 2-Aminopyrimidin ist und das erhaltene Produkt ein Imidazopyrimidin der Formel

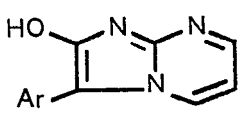

ist, worin Ar eine Arylgruppe bedeutet.

## Claims

1. A process for opening the ring of gem dicyano epoxides of the formula

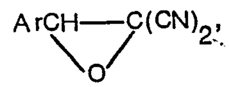

in which Ar denotes an aryl group, wherein a gem dicyano epoxide is reacted with a hydracid under conditions which are usual in the art characterised in that a binucleophilic nitrogen-bearing compound is present in the reaction medium.

2. A process according to claim 1 characterised in that said binucleophilic compound is an amidine and the product obtained is a 4-hydroxy imidazole of the formula:

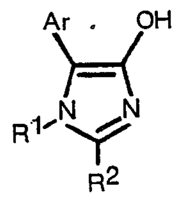

in which Ar denotes an aryl group and $R^1$ and $R^2$ each denote a phenyl group.

3. A process according to claim 1 characterised in that said binucleophilic compound is a 2-amino thiazole and the product obtained is a 6-oxo 5-aryl imidazothiazole.

4. A process according to claim 1 characterised in that said binucleophilic compound is a 2-amino pyridine and the product obtained is a (1,2-a) imidazopyridine of the formula:

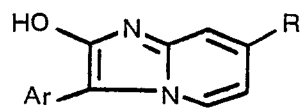

in which Ar denotes an aryl group and R denotes a hydrogen or a methyl.

5. A process according to claim 1 characterised in that said binucleophilic compound is 2-N-benzylamino pyridine and the product obtained is a mesoionic (1,2-a) imidazopyridine of the formula:

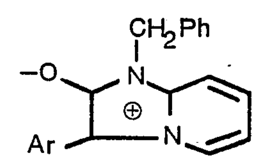

in which Ar denotes an aryl group.

6. A process according to claim 1 characterised in that said binucleophilic compound is 2-amino pyrimidine and the product obtained is an imidazopyrimidine of the formula:

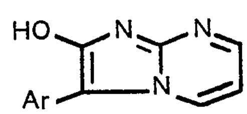

in which Ar denotes an aryl group.